# EUROPEAN PATENT APPLICATION

(11) **EP 4 371 576 A1**
(43) Date of publication of application: **22.05.2024**
(21) Application number: 22841048.6
(22) Date of filing: 19.05.2022
(51) Int. Cl.: A61K 47/54, A61K 33/242, A61P 13/12, B82Y 5/00, B82Y 30/00

(54) **ACETYLCYSTEINE-STABILIZED GOLD NANOCLUSTERS FOR ACUTE KIDNEY INJURY, AND PREPARATION METHOD THEREFOR AND USE THEREOF**

(30) Priority: 12.07.2021 CN 202110786544
(71) Applicant: Shenzhen University, Shenzhen, Guandong 518060 (CN)
(72) Inventor: HUANG, Peng, Shenzhen, Guangdong 518060 (CN); TU, Tianhui, Shenzhen, Guangdong 518060 (CN); ZHANG, Dongyang, Shenzhen, Guangdong 518060 (CN); LIN, Jing, Shenzhen, Guangdong 518060 (CN)
(74) Representative: KIPA AB
(86) International application number: PCT/CN2022/093947
(87) International publication number: WO 2023/284408

(57) **Abstract**

Disclosed are an acetylcysteine-stabilized gold nanoclusters for acute kidney injury, and a prepapration method and an application thereof. The acetylcysteine-stabilized gold nanoclusters (Au NCs-NAC) includes gold nanoclusters and acetylcysteine bound to the surface of the gold nanoclusters. The Au NCs-NAC of the present disclosure includes surface ligand acetylcysteine and gold nanoclusters protected by the surface ligand. The Au NCs-NAC designed in the present disclosure has an ultra-small size, can be effectively enriched in mice kidneys. By clearing a significant amount of reactive oxygen or nitrogen species within the renal tubules, the Au NCs-NAC alleviate and treat glycerol-induced acute kidney injury. Additionally, the Au NCs-NAC possess anti-inflammatory capabilities and exhibits superior therapeutic efficacy compared to acetylcysteine. Furthermore, the Au NCs-NAC demonstrate excellent biocompatibility and biosafety.

## Description

### FIELD OF THE DISCLOSURE

The present disclosure relates to the technical field of biomedical materials, and in particular to an acetylcysteine-stabilized gold nanoclusters (denoted as Au NCs-NAC) for acute kidney injury, and a preparation method and an application thereof.

### BACKGROUND

Acute kidney injury represents a significant health concern for human. Given its high incidence and mortality rates, an estimated 1.7 million deaths occur globally each year. Currently, supportive therapy and kidney transplantation are the most common treatment modalities. Recent studies indicate that the pathogenesis of acute kidney injury is associated with an excess of intracellular reactive oxygen and nitrogen species. Previously, certain small-molecule drugs, such as allopurinol and acetylcysteine, have been proved to be antioxidant to eliminate reactive oxygen species to alleviate acute kidney injury. However, small-molecule drugs exhibit lower bioavailability, greater side effects, and limited efficacy, hindering their clinical applications. Nonetheless, the successful development of antioxidants provides a solid foundation for future treatments of acute kidney injury.

### BRIEF SUMMARY OF THE DISCLOSURE

### Technical Problem

Inventors have discovered that, compared to traditional proteases, metal nano materials offer significant advantages such as low cost, tunable catalytic properties, and scalability. Among metal nano materials, especially gold nanoclusters demonstrate a broad-spectrum capability for eliminating reactive oxygen and nitrogen species. Additionally, they exhibit outstanding anti-inflammatory capabilities. Importantly, the ultra-small size of metal nanoparticles allows for metabolism through the kidneys, offering potential avenues for the treatment of acute kidney injury.

### Technical Solutions

Based on this, the present disclosure developed acetylcysteine-stabilized gold nanoclusters (Au NCs-NAC) for the treatment of acute kidney injury.

Specifically, the present disclosure provides an acetylcysteine-stabilized gold nanocluster (Au NCs-NAC) for acute kidney injury and a preparation method and an application thereof, aiming to solve the technical problems that existing small molecule drugs have low utilization rate, severe side effects and are difficult to be adopted in treating acute kidney injury.

A first aspect of the present disclosure provides an Au NCs-NAC for acute kidney injury, which includes: gold nanoclusters, and acetylcysteine bound to a surface of the gold nanoclusters.

In the present disclosure, the gold nanoclusters and the acetylcysteine are bound through gold-sulfur coordination bonds.

Acetylcysteine (as surface ligand) in the present disclosure can effectively stabilize gold nanoclusters and control the gold nanoclusters to form ultra-small size. Moreover, they all have good water solubility and biosafety, and are not easy to interact with serum proteins, which is beneficial to the circulation of Au NCs-NAC in the blood.

It should be noted that the present disclosure is not limited to adopt acetylcysteine, cysteine can also be used as a surface ligand to stabilize on the surface of gold nanoclusters.

Optionally, the mass ratio of the gold nanoclusters to the acetylcysteine is 1:(1-10).

Optionally, the Au NCs-NAC are spherical cluster particles with a diameter less than or equal to 3 nm.

A second aspect of the present disclosure provides a method for preparing the Au NCs-NAC for acute kidney injury as described above, which includes steps of: mixing chloroauric acid, sodium hydroxide and acetylcysteine in water, stirring and heating to obtain a mixed solution, dialyzing the mixed solution to obtain the Au NCs-NAC.

Optionally, the mass ratio of the chloroauric acid to acetylcysteine is 1:(1-10), such as 1:6.528.

Optionally, a time of the stirring and heating is 1-4 hours.

Optionally, a temperature of the stirring and heating is 25-50 °C.

A third aspect of the present disclosure provides an application of the Au NCs-NAC as described above in preparing a drug for treating acute kidney injury;

Alternatively, provided is an application of the Au NCs-NAC prepared by the above method in preparing a drug for treating acute kidney injury is provided.

### Beneficial Effect

The Au NCs-NAC of the present disclosure includes the surface ligand acetylcysteine and the gold nanoclusters protected by the surface ligand. The surface ligand can effectively stabilize the gold nanoclusters and control the gold nanoclusters to form ultra-small size. Since the Au NCs-NAC of the present disclosure has an ultra-small size, which is below the renal filtration threshold of 5.5 nm, Au NCs-NAC can be effectively enriched in the mice kidneys. By clearing a significant amount of reactive oxygen or nitrogen species within the renal tubules, the Au NCs-NAC alleviate and treat glycerol-induced acute kidney injury. Additionally, the Au NCs-NAC possess anti-inflammatory capabilities and exhibits superior therapeutic efficacy compared to acetylcysteine. Furthermore, the Au NCs-NAC demonstrate excellent biocompatibility and biosafety.

### BRIEF DESCRIPTION OF THE DRAWINGS

Fig. 1 is a synthesis route diagram of Au NCs-NAC in an embodiment of the present disclosure;
Fig. 2 is a TEM image of Au NCs-NAC in an embodiment of the present disclosure;
Fig. 3 is an XPS image of Au NCs-NAC in an embodiment of the present disclosure;
Fig. 4 is a graph comparing the hydroxyl radical eliminating rate of Au NCs-NAC and NAC in embodiments of the present disclosure;
Fig. 5 is a graph comparing the superoxide anion clearance rate of Au NCs-NAC and NAC in embodiments of the present disclosure;
Fig. 6 is a graph comparing the free radical eliminating rate of Au NCs-NAC and NAC in embodiments of the present disclosure;
Fig. 7 is a graph showing the survival rate of renal tubular cells (293T) treated with Au NCs-NAC in an embodiment of the present disclosure;
Fig. 8 is a graph comparing the reactive oxygen species staining of Au NCs-NAC and NAC in renal tubular cells (293T) in an embodiment of the present disclosure;
Fig. 9 is a graph comparing the relative levels of TNF-α of Au NCs-NAC and NAC in renal tubular cells (293T) in embodiments of the present disclosure;
Fig. 10 is a graph comparing the relative levels of IL-6 of Au NCs-NAC and NAC in renal tubular cells (293T) in an embodiment of the present disclosure;
Fig. 11 is a graph showing the blood urea nitrogen content in the serum of mice in different Au NCs-NAC treatment groups in embodiments of the present disclosure;
Fig. 12 is a diagram showing the blood creatinine content in the serum of mice in different Au NCs-NAC treatment groups in embodiments of the present disclosure;
Fig. 13 is a graph showing the survival changes of mice with acute renal failure injected with Au NCs-NAC and phosphate buffer (control) in an embodiment of the present disclosure.

### DETAILED DESCRIPTION OF EMBODIMENTS

The present disclosure provides an Au NCs-NAC for acute kidney injury and a preparation method and an application thereof. In order to make the purpose, technical solution, and effect of the present disclosure clearer, the present disclosure is further described in detail below. It should be understood that the embodiments described here are only used to explain the present disclosure and are not intended to limit the present disclosure.

### Embodiments

An embodiment of the present disclosure provides an Au NCs-NAC for acute kidney injury, which includes: gold nanoclusters, and acetylcysteine bound to the surface of the gold nanoclusters.

Au NCs-NAC in the present embodiment of the present disclosure includes surface ligand acetylcysteine and gold nanoclusters protected by the surface ligand acetylcysteine. The gold nanoclusters and the acetylcysteine are bound through gold-sulfur coordination bonds, specifically the gold element on the surface of the gold nanoclusters forms a coordination bond with the sulfur element in the acetylcysteine, so that the acetylcysteine is bound to the surface of the gold nanoclusters. The acetylcysteine can effectively stabilize the gold nanoclusters and control the gold nanoclusters to form ultra-small size, so that the final obtained Au NCs-NAC has ultra-small size. Moreover, they all have good water solubility and biosafety, and are not easy to interact with serum proteins, which are beneficial to the circulation of Au NCs-NAC in the blood. In the embodiment of the present disclosure, the Au NCs-NAC have an ultra-small size, which is beneficial for effectively reaching the mice kidney, and alleviate and treat acute kidney injury by clearing a significant amount of reactive oxygen or nitrogen species within the renal tubules. Additionally, the Au NCs-NAC possess anti-inflammatory capabilities and exhibits superior therapeutic efficacy compared to single acetylcysteine in the same dose.

It should be noted that the embodiments of the present disclosure are not limited to the gold nanoclusters.

In one embodiment, the mass ratio of the gold nanoclusters to the acetylcysteine is 1:(1-10), such as 1:6.528. The Au NCs-NAC obtained within this ratio range exhibits excellent dispersibility and stability, along with a small size.

In one embodiment, the Au NCs-NAC are spherical particles with a diameter less than or equal to 3 nm. Ultra-small nanoparticles are conducive to reaching the mice kidneys, and ultra-small nanoparticles are conducive to metabolism through the kidneys. Further, the Au NCs-NAC are spherical particles with a diameter of 1-3 nm.

An embodiment of the present disclosure provides a method for preparing the Au NCs-NAC for acute kidney injury as described above, which includes steps of: mixing chloroauric acid, sodium hydroxide, and acetylcysteine in water; stirring and heating to obtain a mixed solution; dialyzing the mixed solution to obtain the Au NCs-NAC.

In one embodiment, the mixed solution is put into a dialysis bag and dialyzed for one day to obtain the Au NCs-NAC.

In one embodiment, the mass ratio of the chloroauric acid to acetylcysteine is 1:(1-10), such as 1:6.528.

In one embodiment, in terms of mass ratio, chloroauric acid : sodium hydroxide : acetylcysteine=1:1.5:6.528. Acetylcysteine can reduce chloroauric acid and control the growth of gold nanoclusters to form ultra-small gold nanoclusters. The use of water as a solvent can ensure good dispersion of the material.

In one embodiment, the time of the stirring and heating is 1-4 hours (such as 2.5 hours).

In one embodiment, the temperature of the stirring and heating is 25-50 °C.

In one embodiment, the chloroauric acid is chloroauric acid trihydrate or chloroauric acid tetrahydrate, but is not limited thereto.

An application of the Au NCs-NAC described in the embodiment of the present disclosure in preparing drugs for treating acute kidney injury.

An application of the Au NCs-NAC prepared by the method described in the embodiment of the present disclosure in preparing drugs for treating acute kidney injury.

The technical solution of the present disclosure is further described below through embodiments.

### Embodiment 1: Synthesis of Au NCs-NAC.

Au NCs-NAC synthesis: chloroauric acid (2 mL, concentration: 20 mg/mL) and NaOH aqueous solution (3 mL, concentration: 0.5 mol/mL) were added to NAC aqueous solution (20 mL, concentration: 80 mmol/mL), and stirred at 37 °C for 2.5 hours. Then, the reacted aqueous solution was dialyzed for two days, and the water was changed every 4 hours. Finally, the resulting solution was stored in a 4 °C refrigerator for later use.

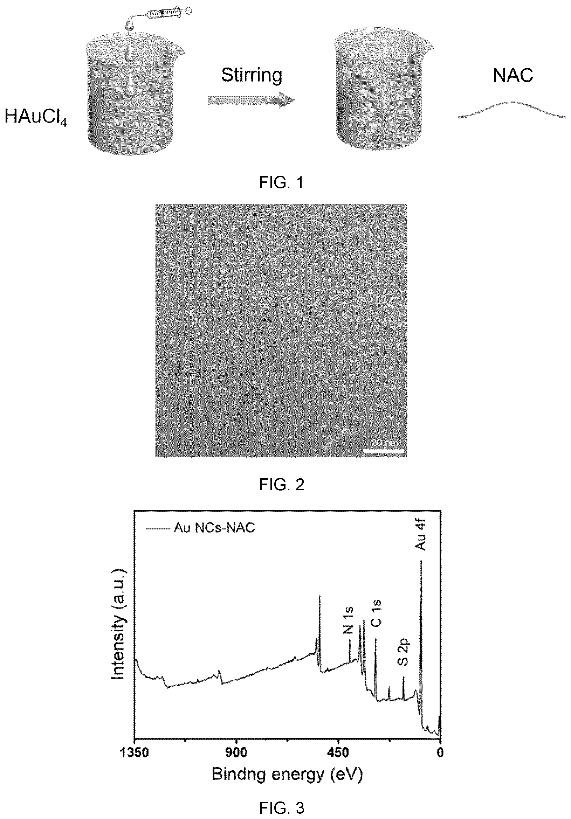

Fig. 1 is a roadmap for the synthesis of Au NCs-NAC, where HAuCl₄ represents chloroauric acid and NAC represents acetylcysteine. The surface ligand acetylcysteine in the Au NCs-NAC can stabilize gold nanoclusters well.

Fig. 2 is the TEM image of the synthesized Au NCs-NAC; Fig. 3 is the XPS image of the synthesized Au NCs-NAC; Fig. 2 and Fig. 3 show that Au NCs-NAC is successfully synthesized and has ultra-small size, and the S element in the XPS demonstrates the successful modification of acetylcysteine.

### Embodiment 2: The ability of Au NCs-NAC to eliminate various reactive oxygen species and hydroxyl radicals.

The efficiency for of Au NCs-NAC in different concentrations (25-100 µg /mL) to eliminate hydroxyl radicals was determined by the hydroxyl radical antioxidant capacity (HORAC) kit (Cell Biolabs, Inc., USA). Testing was performed according to the protocol provided by the manufacturer.

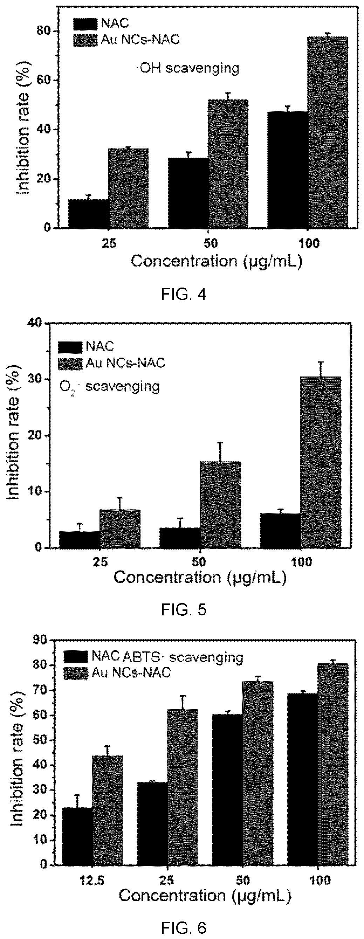

As shown in Fig. 4, the Au NCs-NAC can effectively eliminate hydroxyl radicals with a concentration-dependent property. Moreover, the eliminating efficiency of Au NCs-NAC is significantly superior to that of acetylcysteine alone. At a concentration of 100 µg/mL, Au NCs-NAC can eliminate 77.7% of hydroxyl radicals, while acetylcysteine can only eliminate 47.2%.

The efficiency of Au NCs-NAC in removing superoxide anions at different concentrations (25-100 µg/mL) was determined using the SOD assay kit (Sigma-Aldrich, USA). The test was conducted according to the instructions provided by the manufacture.

As shown in Fig. 5, Au NCs-NAC can effectively eliminate superoxide anions with a concentration-dependent property. Furthermore, the superoxide anion eliminating efficiency of Au NCs-NAC is significantly superior to that of acetylcysteine alone. At a concentration of 100 µg/mL, Au NCs-NAC can eliminate 30.4% of superoxide anions, while acetylcysteine alone can only eliminate 6%.

The test for eliminating ABTS (2,2'-azino-bis ( 3-ethylbenzothiazoline-6-sulfonic acid) ) free radicals by Au NCs-NAC.

The free radicals eliminating ability of Au NCs-NAC was tested using the ABTS free radical cation discoloration method. ABTS (7 mM) was dissolved in water, and 2.45 mM potassium persulfate was added to react for 12 hours to produce ABTS free radical cations (•ABTS+). Then, the absorbance values of the pure •ABTS+ solution (AB) and the mixed solution of different concentrations (12.5-100 µg/mL) of Au NCs-NAC and •ABTS+ were measured at 734 nm. The formula for calculating ABTS eliminating efficiency is [(AB - AP)/AB] * 100. All measurements were performed in triplicate.

As shown in Fig. 6, Au NCs-NAC can effectively eliminate free radicals with a concentration-dependent property. Additionally, the ABTS eliminating efficiency of Au NCs-NAC is significantly superior to that of acetylcysteine alone. At a concentration of 100 µg/mL, Au NCs-NAC can eliminate 80.6% of •ABTS+, while acetylcysteine alone can only eliminate 68.9%.

### Embodiment 3: Au NCs-NAC cytotoxicity and protection of kidney cells by eliminating various reactive oxygen species/reactive nitrogen species, and the standard MTT method was used to evaluate the effect of Au NCs-NAC on the survival rate of 293T renal embryonic cells. Furthermore, ELISA (enzyme-linked immunosorbent assay) kits for TNF-α and IL-6 are adopted to evaluate the levels of inflammatory factors in the cells.

293T cells were seeded into a 96-well plate at a density of 1×104 per well and incubated at 37 °C and 5% CO₂ for 12 h. Next, the old culture medium in the 96-well plate was aspirated, and culture medium solutions containing Au NCs-NAC at different concentrations were added. After culturing for another 20 h, the old culture medium in the 96-well plate was aspirated, and 100 µL MTT culture medium solution (0.8 mg/mL) was added to each well, and the cells were cultured for another 4 h. The remaining culture medium in the 96-well plate was aspirated, and 150 µL DMSO solution was added to each well. After gently shaking, the OD value of each well was detected on a Synergy H1 microplate reader (detection wavelength was 570 nm). The following formula was adopted to calculate the cell survival rate: Cell viability (%) = (OD570 of the sample / OD570 of the blank) × 100%.

For in vitro anti-inflammatory studies, both macrophages and 293T renal embryonic cells were seeded into 96-well plates. Then, 293T renal embryonic cells were treated with 400 ng/mL lipopolysaccharide (LPS) for 1 h. Then, the supernatant of 293T renal embryonic cell culture was transferred to a 96-well plate to culture RAW264.7 macrophages overnight. Finally, the TNF-α/IL-6 ELISA kit was used to detect the concentrations of TNF-α and IL-6 in the RAW264.7 macrophage supernatant.

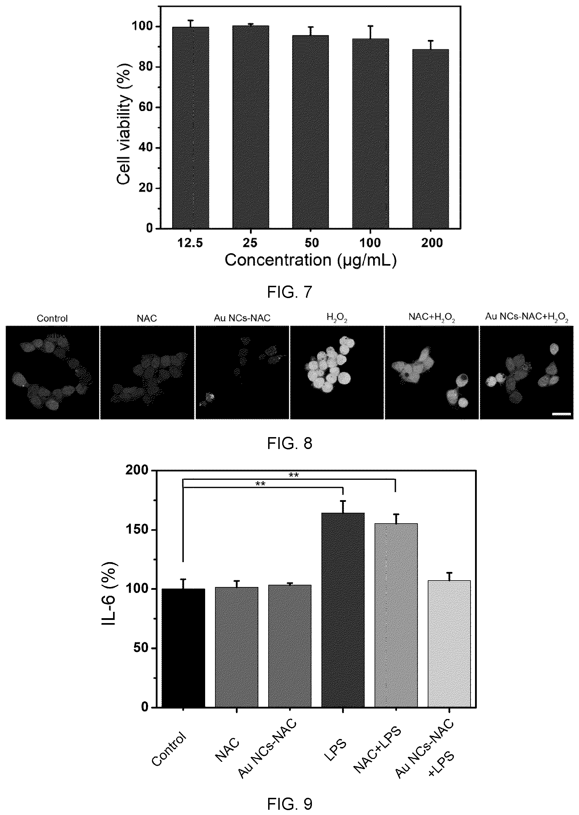

As shown in Fig. 7, it is the cell survival rate of 293T renal embryonic cells treated by the synthesized Au NCs-NAC, the cells still maintain a survival rate of more than 80% even reaching the maximum concentration of Au NCs-NAC of 200 µg/mL. It shows that the Au NCs-NAC in the present embodiment has low cytotoxicity.

Taking Au NCs-NAC as an example, 293T cells were treated with Au NCs-NAC (100 µg/mL) 4 hours in advance, and then medium containing 2 mM hydrogen peroxide was added. The reactive oxygen species probe DCFH-DA was then used respectively (Fig. 8), imaging was performed after washing using a laser confocal microscope. As shown in Fig. 8, compared with cells stimulated by hydrogen peroxide, the reactive oxygen species fluorescence in cells treated with Au NCs-NAC is significantly weakened and is close to that of cells in the control group. This shows that Au NCs-NAC can effectively remove reactive oxygen/nitrogen in cells and thereby protect cells. In addition, as shown in Figs. 9-10, Au NCs-NAC can effectively reduce the production of inflammatory factors (TNF-α and IL-6), which are produced by macrophages due to LPS stimulation, to be close to normal cell levels. However, equivalent doses of acetylcysteine alone are not able to achieve the same.

### Embodiment 4: Au NCs-NAC treatment of acute kidney injury and biosafety evaluation.

All experimental operations were performed in accordance with the animal use and care system approved by the Clinical Center Animal Care and Use Committee. Female athymic mice (six weeks, 20-25 g) were injected with 8 mL/kg 50% glycerol solution intramuscularly into the hind legs to establish a mice model of acute renal failure. After 2 hours, the small molecule drug acetylcysteine or Au NCs-NAC was injected.

Mice were randomly divided into 5 groups: (1) healthy mice injected with phosphate buffer; (2) healthy mice injected with Au NCs-NAC; (3) glycerol-induced acute renal failure mice injected with phosphate buffer; (4) glycerol-induced acute renal failure mice injected with Au NCs-NAC; (5) glycerol-induced acute renal failure mice injected with acetylcysteine in the same dose of Au NCs-NAC. Healthy mice and mice with glycerol-induced acute renal failure were euthanized 24 hours later. The blood of the mice was centrifuged to obtain serum, and the creatinine and blood urea nitrogen contents were measured. The phosphate buffer used for injection was 100 µL, the injection dose of Au NCs-NAC was 4 mg/kg, and the injection dose of acetylcysteine was 4 mg/kg.

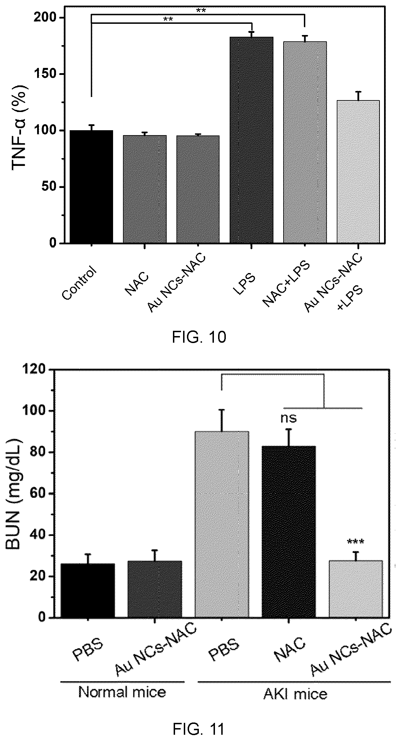

As shown in Figs. 11-12, there is no significant change in the creatinine and blood urea nitrogen contents of healthy mice injected with Au NCs-NAC. While the creatinine and blood urea nitrogen levels of acute renal failure mice injected with Au NCs-NAC are significantly lower than those of mice injected only with phosphate buffer, and are close to the levels of healthy mice. On the other hand, the same dose of acetylcysteine cannot effectively reduce the two indicators. This shows that Au NCs-NAC can effectively alleviate and treat acute renal failure, and has better therapeutic effect than the small molecule drug acetylcysteine alone.

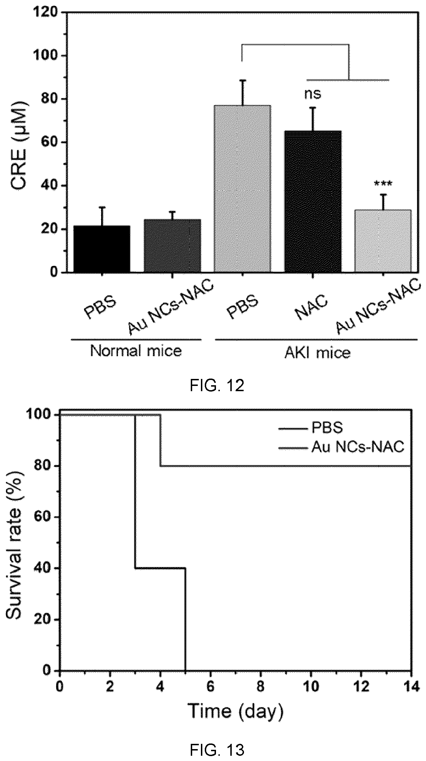

In addition, mice with acute renal failure were injected with phosphate buffer and Au NCs-NAC, and the survival of the mice within fifteen days was recorded. As shown in Fig. 13, compared with the control group, the survival rate of mice injected with Au NCs-NAC is significantly improved.

### Industrial Applicability

In summary, the present disclosure enables the large-scale production of ultra-small nanometer-sized particles Au NCs-NAC through a simple synthesis method. The Au NCs-NAC can effectively clear various reactive oxygen species/reactive nitrogen species, demonstrating a broad-spectrum capability of eliminating reactive oxygen/reactive nitrogen superior to that of acetylcysteine alone. Moreover, it exhibits low cytotoxic towards 293T renal cells, with cell survival rates consistently exceeding 80% after 24 hours of co-culture. Additionally, Au NCs-NAC protects cells from hydrogen peroxide stimulation by eliminating excess intracellular reactive oxygen/reactive nitrogen. Furthermore, it significantly reduces the production of inflammatory factors, a feat unattainable under equivalent dose condition with acetylcysteine alone. Notably, Au NCs-NAC demonstrates promising therapeutic effects in mice with glycerol-induced acute renal failure, surpassing the efficacy of equivalent doses of acetylcysteine alone. More important, Au NCs-NAC exhibits excellent biocompatibility and biosafety.

It should be understood that the application of the present disclosure is not limited to the above embodiments. Those skilled in the art can make improvements or changes based on the above descriptions. All these improvements and changes should fall within the protection scope of the appended claims of the present disclosure.

## Claims

1. An acetylcysteine-stabilized gold nanoclusters for acute kidney injury, wherein the acetylcysteine-stabilized gold nanoclusters comprise: gold nanoclusters, and acetylcysteine bound to a surface of the gold nanoclusters.

2. The acetylcysteine-stabilized gold nanoclusters for acute kidney injury according to claim 1, wherein the gold nanoclusters and the acetylcysteine are bound through gold-sulfur coordination bonds.

3. The acetylcysteine-stabilized gold nanoclusters for acute kidney injury according to claim 1, wherein the mass ratio of the gold nanoclusters to the acetylcysteine is 1:(1-10).

4. The acetylcysteine-stabilized gold nanoclusters for acute kidney injury according to claim 1, wherein the acetylcysteine-stabilized gold nanoclusters are spherical cluster particles with a diameter less than or equal to 3 nm.

5. A method for preparing the acetylcysteine-stabilized gold nanoclusters for acute kidney injury according to any one of claims 1-4, wherein the method comprises: mixing chloroauric acid, sodium hydroxide and acetylcysteine in water, stirring and heating to obtain a mixed solution, dialyzing the mixed solution to obtain the Au NCs-NAC.

6. The method for preparing the acetylcysteine-stabilized gold nanoclusters for acute kidney injury according to claim 5, **characterized in that** the mass ratio of the chloroauric acid and acetylcysteine is 1: (1-10).

7. The method for preparing the acetylcysteine-stabilized gold nanoclusters for acute kidney injury according to claim 5, wherein a time of the stirring and heating is 1-4 hours.

8. The method for preparing the acetylcysteine-stabilized gold nanoclusters for acute kidney injury according to claim 5, wherein a temperature of the stirring and heating is 25-50 °C.

9. A use of the acetylcysteine-stabilized gold nanoclusters according to any one of claims 1-4 in preparing drugs for treatment of acute kidney injury;
or, a use of the acetylcysteine-stabilized gold nanocluster prepared by the method according to any one of claims 5-8 in preparing drugs for treatment of acute kidney injury.
